# EUROPÄISCHE PATENTSCHRIFT

(11) **EP 2 371 316 B1**
(45) Veröffentlichungstag und Bekanntmachung des Hinweises auf die Patenterteilung: **07.11.2018**
(21) Anmeldenummer: 11159304.2
(22) Anmeldetag: 23.03.2011
(51) Int. Cl.: A61B 18/14, A61B 17/29, A61B 17/00

(54) **Chirurgisches Instrument, insbesondere elektrochirurgisches Instrument**
Surgical instrument, in particular electro-surgical instrument
Instrument chirurgical, notamment instrument électrochirurgical

(30) Priorität: 01.04.2010 DE 102010016291
(43) Veröffentlichungstag der Anmeldung: 05.10.2011
(73) Patentinhaber: Erbe Elektromedizin GmbH, 72072 Tübingen (DE)
(72) Erfinder: Mitzlaff, Lothar, 8600-531, Lagos (PT); Krause, Ralf, 71157, Hildrizhausen (DE)
(74) Vertreter: Bohnenberger, Johannes

(56) Entgegenhaltungen:
- WO-A1-01/66025
- DE-B4- 10 084 618
- US-A- 5 190 560
- US-A- 5 562 655
- US-A1- 2005 256 533
- US-B2- 7 628 791

## Beschreibung

Die Erfindung betrifft ein chirurgisches Instrument, insbesondere elektrochirurgisches Instrument, nach Anspruch 1.

Chirurgische Instrumente, die mehrere Funktionen zusammenfassen, sind im Stand der Technik bekannt. Bei derartig kombinierten Instrumenten entfällt ein Wechsel zwischen den zu einem bestimmten Zeitpunkt benötigten "Einzelinstrumenten". Beispielsweise laparoskopische Operationen, bei denen üblicherweise ein Instrumentenwechsel mit einem aufwändigen Herausziehen und Einführen in den Trokar verbunden ist, können so effizienter durchgeführt werden. Bekannt sind beispielsweise kombinierte Instrumente zur Gefäßversiegelung und zum Schneiden von Gewebe.

Eine häufige Anwendung für derartige Instrumente ist das Absetzen von Gewebe, das entnommen werden soll. Beim Absetzen wird in einem ersten Schritt Gewebe gegriffen und koaguliert. In einem zweiten Schritt wird der koagulierte Abschnitt mit einem Schneidinstrument durchtrennt. Die Koagulation erfolgt dabei, um Blutgefäße zu verschließen und eine Blutung beim Schneiden zu verhindern. Diese Schritte werden so oft wiederholt, bis das Gewebebündel vom Körper abgetrennt ist. Derartige Verfahren sind Standard bei Krebserkrankungen, beispielsweise bei einer Hysterektomie, bei der die Gebärmutter entfernt wird.

Ein solches Instrument benötigt eine erste Funktionseinheit für das Greifen und eine zweite Funktionseinheit für das Schneiden des Gewebes, wobei eine Mechanik zur Übertragung von Kraft bzw. Bewegung vorgesehen sein muss. Die entsprechende Kraft kann vom Anwender durch Bedienelemente an einem Handgriff, beispielsweise einen Handabzug oder einen Fingerabzug, aufgewendet werden.

Üblicherweise sind bei chirurgischen Instrumenten gemäß dem Stand der Technik mehrere Betätigungselemente vorgesehen, wobei jedes Betätigungselement einer Funktionseinheit zugeordnet ist. Dadurch können die Funktionseinheiten getrennt bedient werden. Beispielsweise können ein Handabzug zum Greifen, ein Fingerabzug zum Schneiden und ein separater Koagulationsschalter vorgesehen sein. Die separate Ausbildung der Betätigungselemente wird als nachteilig empfunden, da die Operation oftmals unterbrochen werden muss, weil beispielsweise ein flüssiges Greifen und Schneiden in einem Zug nicht möglich ist. Weiterhin ist der Benutzer eingeschränkt, beispielsweise wenn der Zeigefinger zum Schneiden gebraucht wird und nicht mehr für die Aktivierung des Koagulationsvorgangs zur Verfügung steht. Außerdem führen die verschiedenen Betätigungselemente zu Mehrkosten und vergleichsweise "sperrigen" Instrumenten. Insgesamt gesehen wird das Instrument in der Anwendung unübersichtlicher.

Weiterhin sind im Stand der Technik chirurgische Instrumente bekannt, bei denen zwei verschiedene Funktionen, konkret Greifen und Schneiden von Gewebe, mit nur einem Betätigungselement gesteuert werden können. Beispielsweise beschreibt die US 7 628 791 B2 ein elektrochirurgisches Instrument zum Koagulieren und Schneiden von Gewebe. Drei verschiedene Funktionen, konkret das Greifen, Koagulieren und Schneiden des Gewebes, werden mit nur einem Betätigungselement gesteuert. Wird der vorgesehene Handabzug um eine erste Wegstrecke durchgezogen, schließt sich das Maulteil, wird er weiter durchgezogen, wird ein Koagulationsschalter betätigt, wird er noch weiter durchgezogen, wird eine Klinge durch das geklemmte Gewebe geführt. Über den gesamten Bewegungsbereich muss vom Anwender eine Kraft ausgeübt werden, um das Gewebe festzuhalten, wobei die Gegenkraft einer Feder überwunden wird. Zur Arretierung einer Greifposition werden in der US 7 628 791 B2 zwar Rasteinrichtungen vorgeschlagen, diese ermöglichen jedoch nur eine Verrastung bevor der Schneidevorgang einsetzt. Während des Schneidevorgangs ist eine entsprechende Verrastung nicht möglich und wäre auch nachteilig, da dies bei der bekannten Vorrichtung zur Folge hätte, dass das Schneidemesser nicht zurückbewegt werden könnte. Insgesamt ist der Anwender bei der Bedienung des Instruments gemäß US 7 628 791 B2 vergleichsweise eingeschränkt, beispielsweise muss während des Schneidens des Gewebes darauf geachtet werden, dass das Gewebe ausreichend gehalten wird.

WO0166025 und US5562655 sind zusätzliche Dokumente des Stands der Technik. Es ist Aufgabe der vorliegenden Erfindung, ein chirurgisches Instrument mit mindestens zwei Funktionseinheiten mit einfacher und sicherer Handhabung vorzuschlagen.

Diese Aufgabe wird durch ein chirurgisches Instrument, insbesondere elektrochirurgisches Instrument, gemäß Anspruch 1 gelöst.

Die Aufgabe wird insbesondere durch ein chirurgisches Instrument, insbesondere elektrochirurgisches Instrument, mit einem Betätigungselement zur Betätigung wenigstens einer ersten und einer zweiten Funktionseinheit gelöst, wobei das Betätigungselement innerhalb eines Bewegungsbereichs rotatorisch bewegbar ist, wobei durch eine Bewegung innerhalb eines ersten Teilbereichs des Bewegungsbereichs die erste Funktionseinheit betätigbar ist und durch eine Bewegung innerhalb eines zweiten Teilbereichs des Bewegungsbereichs die zweite Funktionseinheit betätigbar ist, wobei eine Umkopplungsvorrichtung vorgesehen ist derart, dass in Folge einer Bewegung des Betätigungselements durch einen Umkopplungsbereich zwischen dem ersten und zweiten Teilbereich eine Wirkverbindung zwischen erster Funktionseinheit und Betätigungselement unterbrochen wird und eine Wirkverbindung zwischen zweiter Funktionseinheit und Betätigungselement hergestellt wird. Ein wesentlicher Punkt der Erfindung liegt darin, dass aufgrund der vorgesehenen Umkopplungsvorrichtung eine Bewegung innerhalb des ersten Teilbereichs keine Auswirkung auf die zweite Funktionseinheit hat und umgekehrt, dass eine Bewegung innerhalb des zweiten Teilbereichs keine Auswirkung auf die erste Funktionseinheit hat. Der Anwender kann sich somit auf die Durchführung der ersten bzw. zweiten Funktion konzentrieren, ohne Rücksicht auf eine jeweils andere Funktion nehmen zu müssen. Zur Betätigung der Funktionseinheiten ist dennoch nur ein Betätigungselement notwendig. Insgesamt ist die Bedienbarkeit des chirurgischen Instruments gegenüber dem Stand der Technik verbessert.

Im Umkopplungsbereich, ggf. in einem Unterbereich des Umkopplungsbereiches, wird eine Wirkverbindung zwischen Betätigungselement und erster und zweiter Funktionseinheit hergestellt oder unterbrochen . Vorzugsweise ist mindestens eine Arretiervorrichtung vorgesehen derart, dass die erste Funktionseinheit oder eine Teilkomponente dieser bei einer Bewegung des Betätigungselements innerhalb des zweiten Teilbereichs arretiert ist und gegebenenfalls umgekehrt, dass die zweite Funktionseinheit oder eine Teilkomponente dieser bei einer Bewegung des Betätigungselements innerhalb des ersten Teilbereichs arretiert ist. Die Arretiervorrichtung kann Bestandteil der Umkopplungsvorrichtung sein. Eine derartige Arretierung erleichtert weiterhin die Bedienung des chirurgischen Instrumentes. Ist die erste Funktionseinheit beispielsweise eine Festhaltevorrichtung bzw. Greifvorrichtung für ein Gewebe, so ist gewährleistet, dass die Festhaltevorrichtung hinsichtlich ihrer ausgeübten Kraft und/oder ihrer Position konstant ist.

In einer bevorzugten Ausgestaltung umfasst die Arretiervorrichtung bzw. die Umkopplungsvorrichtung eine Kniehebeleinrichtung. Die Kniehebeleinrichtung kann derart ausgebildet sein, dass eine Arretierung bzw. Umkopplung bei Überschreiten eines Totpunktes stattfinden kann. Der Totpunkt definiert in diesem Fall den Umkopplungsbereich. Im Allgemeinen kann der Umkopplungsbereich punktförmig sein, wobei die Umkopplung bei Überschreiten des entsprechenden Umkopplungspunktes stattfinden kann. Die Arretierung bzw. Umkopplung mit Hilfe einer Kniehebeleinrichtung ist konstruktiv besonders einfach und kann auf einfache Art und Weise erfolgen.

In einer konkreten Ausführungsform umfasst die Umkopplungsvorrichtung bzw. Arretiervorrichtung eine Führungsschiene, in der das Betätigungselement, insbesondere (im Wesentlichen) in einer Bewegungsebene, führbar ist. Die Führungsschiene kann derart ausgebildet sein, dass eine Umkopplung bzw. Arretierung, gegebenenfalls im Zusammenwirken mit weiteren Komponenten, durch eine Bewegung des Betätigungselements innerhalb der mindestens einen Führungsschiene ausgelöst werden kann. Eine derartige Führungsschiene erleichtert die Umkopplung bzw. Arretierung und somit die Bedienung des chirurgischen Instruments.

Vorzugsweise ist die Führungsschiene V- oder U-förmig ausgebildet, wobei ein erster V-/U-Schenkel kürzer ist als ein zweiter V-/U-Schenkel. Vorzugweise ist die Führungsschiene derart ausgebildet, dass die Arretierung realisiert ist, wenn die Führungsschiene innerhalb des ersten, kürzeren V-/U-Schenkels angeordnet ist. Weiter vorzugsweise kann die Führungsschiene derart ausgebildet sein, dass die Arretierung aufgehoben ist, wenn das Betätigungselement in den zweiten, längeren V-/U-förmigen Schenkel angeordnet bzw. überführt ist. Gegebenenfalls kann die Führungsschiene derart ausgebildet sein, dass bei einer Überführung vom einen Schenkel in den Anderen gleichzeitig die Umkopplung erfolgt. Derartige Führungsschienen sind konstruktiv einfach und erleichtern die Bedienung.

In einer konkreten Ausführungsform ist die Führungsschiene insbesondere integraler Bestandteil eines Gehäuseelementes, vorzugsweise einer Griffschale. Dadurch kann ein Bauteil eingespart werden, was den Kostenaufwand senkt.

Vorzugsweise umfasst die erste/zweite Funktionseinheit eine Schneidvorrichtung, wie beispielsweise eine mechanische Schneidvorrichtung oder eine Laserschneidvorrichtung, und/oder eine Festhaltevorrichtung bzw. Greifvorrichtung und/oder eine Koagulationsvorrichtung, insbesondere HF-Koagulationsvorrichtung, und/oder eine Wasserstrahlvorrichtung, insbesondere eine Wasserstrahlschneidvorrichtung, und/oder eine optische Einrichtung, wie beispielsweise eine Kamera oder eine Beleuchtungseinrichtung, und/oder eine Absaugvorrichtung, und/oder eine Spülvorrichtung, und/oder eine Kryosonde, und/oder eine Biopsievorrichtung, wie beispielsweise eine Biopsiesonde oder einen Biopsiegreifer, und/oder eine vorzugsweise monopolare Schneidelektrode. Weiter vorzugsweise umfasst die erste Funktionseinheit eine Festhaltevorrichtung und die zweite Funktionseinheit eine Schneidvorrichtung.

Weiter vorzugsweise sind mindestens eine Zahnstange und mindestens ein Zahnrad zur Wirkübertragung zwischen dem Betätigungselement und mindestens einer Funktionseinheit vorgesehen.

Vorzugsweise ist das Zahnrad in Eingriff mit einer Zahnreihe bringbar, die vorzugsweise mit einem/dem Gehäuseelement, weiter vorzugsweise einer/der Griffschale, verbunden ist und insbesondere integral mit dem Gehäuseelement bzw. der Griffschale ausgebildet ist, derart, dass eine Rotation des Zahnrades eine translatorische Verschiebung der Zahnstange gegenüber dem Gehäuseelement bzw. der Griffschale bewirkt. Dadurch wird die Kraftübertragung weiter vereinfacht, wobei weitere Bauteile eingespart werden können, was die Kosten senkt.

Erfindungsgemäß ist die erste/zweite Funktionseinheit, insbesondere die Schneidvorrichtung und/oder die Festhaltevorrichtung durch Betätigen des Betätigungselementes, vorzugsweise in jeder Position innerhalb des entsprechenden Teilbereichs, in zwei verschiedene Richtungen bewegbar. Dies erleichtert weiter die Bedienung des chirurgischen Instruments.

In einer konkreten Ausführungsform ist mindestens eine dritte Funktionseinheit, beispielsweise eine Koagulationseinrichtung, durch ein weiteres (separates) Betätigungselement betätigbar. Dadurch kann eine weitere Funktion, beispielsweise das Koagulieren, unabhängig von der Betätigung der ersten oder zweiten Funktionseinheit erfolgen. Dies erleichtert die Bedienung.

Weiterhin kann das Betätigungselement zur Vereinfachung der Bedienung einen Handabzug umfassen.

Das Betätigungselement kann um mindestens zwei voneinander beabstandete Rotationsachsen rotierbar sein. Durch eine derartige Veränderung der Rotationsachse kann dasselbe Betätigungselement besonders einfach verschiedene Komponenten mit Kraft beaufschlagen.

In einer konkreten Ausführungsform ist das Betätigungselement zur Betätigung der ersten Funktionseinheit um eine erste Achse und zur Betätigung der zweiten Funktionseinheit um eine zweite Achse rotierbar. Die Umkopplung kann die Verschiebung der Rotationsachsen umfassen. Die Betätigung der einzelnen Funktionseinheiten ist in diesem Fall nur mit geringem Konstruktionsaufwand verbunden.

Vorzugsweise liegt eine/die zweite Rotationsachse auf einer zentralen Achse des Kniehebelgelenks. Alternativ oder zusätzlich kann eine/die erste/zweite Rotationsachse innerhalb der Führungsschiene, insbesondere innerhalb des kürzeren U-/V-Schenkels angeordnet sein. Gegebenenfalls kann während der Rotation eine zumindest geringe Translation stattfinden, so dass die Rotationsachse sich während der Rotation verschiebt. Die Verschiebung ist in diesem Fall vorzugsweise kleiner als 5 mm, weiter vorzugsweise kleiner als 3 mm. Wenn das Betätigungselement um die zentrale Achse des Kniehebelgelenks rotierbar ist, kann eine Betätigung der entsprechenden Funktionseinheit besonders einfach erfolgen.

Erfindungsgemäß ist das Betätigungselement rotierbar und kann, insbesondere über das Kniehebelgelenk, eine Gleiteinrichtung translatorisch antreiben. Die Gleiteinrichtung kann eine bestimmte Funktion auslösen, insbesondere über Verbindungselemente, wie beispielsweise eine Schubstange, und verringert den Aufwand bei der Herstellung.

In einer konkreten Ausführungsform ist mindestens ein Federelement derart vorgesehen, dass eine Kraft des Betätigungselements über das Federelement an die erste/zweite Funktionseinheit übertragen werden kann. Durch die Zwischenschaltung des Federelements kann die Kraft des Betätigungselementes abgepuffert oder gespeichert werden. Weiterhin kann ein derartiges Federelement die erste/zweite Funktionseinheit mit Druck beaufschlagen, wenn das erste/zweite Funktionselement in einer arretierten Position ist. Beispielsweise wenn die Funktionseinheit eine Festhaltevorrichtung ist, kann das Gewebe zuverlässig und mit konstanter Kraft festgehalten werden, ohne dass der Anwender dazu tätig werden muss.

Vorzugsweise ist mindestens ein Federelement, vorzugsweise eine Zugfeder, vorgesehen derart, dass das Federelement bei einer Verschiebung der Funktionseinheit eine Rückstellkraft auf diese ausübt. Ein derartiges Federelement erlaubt es auf einfache Weise, dass die entsprechende Funktion, beispielsweise das Festhalten des Gewebes, ohne ein aktives Halten des Anwenders gelöst bzw. deaktiviert wird.

Vorzugsweise ist das Betätigungselement derart ausgebildet, dass die Bewegung innerhalb des ersten Teilbereiches und/oder des zweiten Teilbereiches und/oder des Umkopplungsbereiches in einer Ebene durchführbar ist. Dadurch wird der Aufwand bei der Anwendung reduziert.

Erfindungsgemäß ist die Umkopplungsvorrichtung derart reversibel ausgebildet, dass eine Bewegungsumkehr zu einer in den Ausgangszustand zurückführenden Umkopplung führt. Vorzugsweise ist
die Arretiervorrichtung derart reversibel ausgebildet, dass eine Bewegungsumkehr des Betätigungselementes zu einer in den Ausgangzustand zurückführenden Umkopplung bzw. zu einer Aufhebung der Arretierung führt. Dies erleichtert die Handhabung des Instrumentes.

Insgesamt ermöglicht das chirurgische Instrument ein ergonomisches und effektives Arbeiten. Durch die (mechanische) Entkopplung der Teilbereiche werden keine Kräfte auf die jeweils andere Funktionseinheit oder das Betätigungselement übertragen. Beispielsweise muss der Anwender zwar zunächst zum Greifen vom Gewebe (allgemein zum Betätigen der ersten Funktionseinheit) Kraft aufbringen. Nach der Arretierung kann er aber im zweiten Teilbereich unabhängig von dieser Schließkraft (Kraft) arbeiten. Die Funktionen können mit denselben "Körperteilen" durchgeführt werden (beispielsweise Mittel- und/oder Ring- und/oder kleiner Finger), wobei andere "Körperteile" zur Bedienung frei bleiben. Beispielsweise kann der Zeigefinger bei der Bedienung einer Schneidvorrichtung frei bleiben, so dass er, analog zum Abzug einer Pistole, zum Bedienen eines Koagulationsschalters eingesetzt werden kann.

Weitere Vorteile ergeben sich bei der Verwendung der Erfindung in einem kombinierten Koagulations- und Schneidinstrument. Das Schließen eines Maulteils einer Greifvorrichtung und das Schneiden durch eine Schneidvorrichtung können beide in Schließrichtung des Handabzugs erfolgen. Das Zurückführen eines Messers in die Ausgangsstellung, das Entriegeln und das Öffnen des Maulteils können in die Gegenrichtung erfolgen. Dies ermöglicht eine schnelle Abfolge der Schritte: Greifen, Koagulieren und Schneiden. Auch ein vergleichsweise schnelles Schneiden von Gewebe ohne Koagulieren, mit nur einem Betätigungselement, analog einer Schere, ist so möglich. Das Kniehebelsystem ermöglicht die Zurückführung des Messers, die Entriegelung der Arretierung und das Öffnen des Maulteils in einer Bewegungsrichtung. Koagulationen können so mehrfach und vergleichsweise sicher, auch ohne Schnitt, ausgeführt werden. Das Instrument bietet eine gute Präparierfunktion, da das Maulteil aktiv vom Anwender geöffnet werden kann und nicht zwangsläufig von einer Feder abhängt.

Für das Koagulieren kann ein separates Bedienelement vorgesehen sein, ggf. ohne eine zugeordnete mechanische Funktionseinheit. Zum Koagulieren kann ein elektrochirurgischer Generator vorgesehen sein, der einen HF-Strom erzeugt, der über das Instrument durch das gegriffene Gewebe geleitet wird. Der HF-Strom kann in einen monopolaren oder bipolaren Betrieb appliziert werden. Das Signal zur Koagulation erfolgt im Regelfall durch einen Fingerschalter, gegebenenfalls auch am Handgriff oder durch einen Fußschalter.

Weitere Ausführungsformen ergeben sich aus den Unteransprüchen.

Nachfolgend wird die Erfindung auch hinsichtlich weiterer Nachteile und Vorteile anhand von einem Ausführungsbeispiel beschrieben, das anhand der Abbildungen näher erläutert wird.

Hierbei zeigen:
- Fig. 1: ein elektrochirurgisches Instrument in einer Seitenansicht gemäß einer ersten Ausführungsform der Erfindung;
- Fig. 2: das elektrochirurgische Instrument in einer ersten Innenansicht;
- Fig. 3: das elektrochirurgische Instrument in einer ersten Schnittansicht;
- Fig. 4: das elektrochirurgische Instrument in einer zweiten Innenansicht;
- Fig. 5: das elektrochirurgische Instrument in einer zweiten Schnittansicht;
- Fig. 6: das elektrochirurgische Instrument in einer dritten Innenansicht;
- Fig. 7: das elektrochirurgische Instrument in einer dritten Schnittansicht;
- Fig. 8: das elektrochirurgische Instrument in einer vierten Innenansicht;
- Fig. 9: das elektrochirurgische Instrument in einer vierten Schnittansicht;
- Fig. 10: eine Griffschale des elektrochirurgischen Instrumentes in einer Innenansicht.

In der nachfolgenden Beschreibung werden für gleiche und gleich wirkende Teile dieselben Bezugsziffern verwendet.

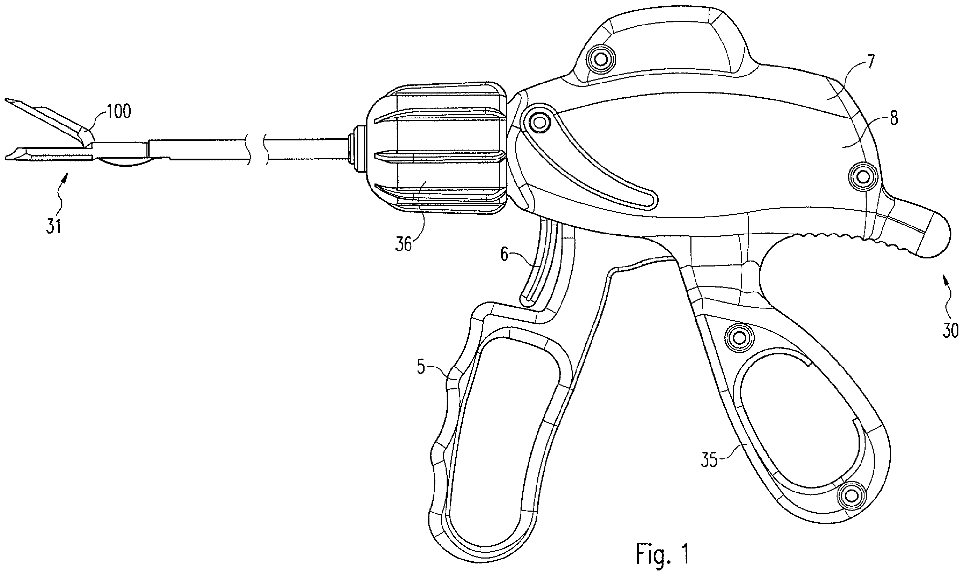

Fig. 1 zeigt ein elektrochirurgisches Instrument in einer Seitenansicht, das zum Greifen von Gewebe (erste Funktion), zum Koagulieren (dritte Funktion) und zum Schneiden (zweite Funktion) ausgebildet ist. Das Greifen und Schneiden von Gewebe erfolgt durch ein gemeinsames Betätigungselement 5, konkret einen Handabzug. Die Koagulation wird über ein zweites Betätigungselement 6, konkret einen Fingerabzug gesteuert. Der Handabzug 5 und der Fingerabzug 6 sind gegenüber einem Griffgehäuse 7, das zwei gegenüberliegende Griffschalen 8 aufweist, beweglich angeordnet.

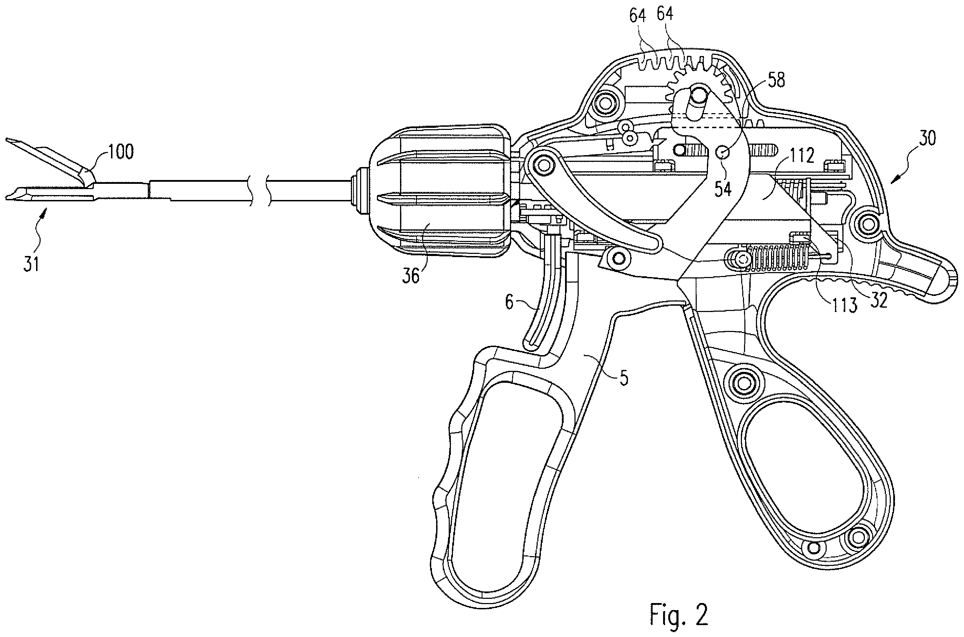

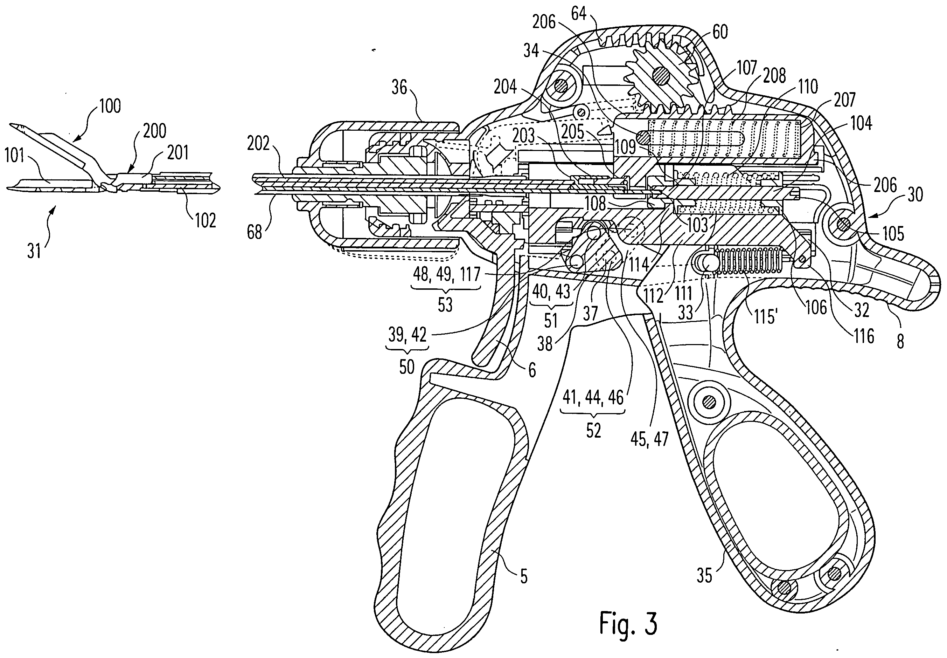

Die Fig. 2 und 3 zeigen Ansichten des elektrochirurgischen Instrumentes, wobei der Handabzug 5 in einer ersten Position angeordnet ist. Der Handabzug 5 dient zur Betätigung sowohl einer Greifvorrichtung 100 (als erste Funktionseinheit) als auch einer Schneidvorrichtung 200 (als zweite Funktionseinheit).

Die Greifvorrichtung 100 umfasst ein Maulteil 101, an dem eine Greifvorrichtungsstange oder ein Greifvorrichtungsdraht 102 angeordnet ist, so dass eine Zugbewegung in Richtung eines proximalen Endes 30 eine Schließbewegung des Maulteils 101 zur Folge hat und umgekehrt eine Schubbewegung in Richtung eines distalen Endes 31 eine Öffnung des Maulteils 101. Hier und im Folgenden soll unter einem proximalen Element/Abschnitt ein Element/Abschnitt verstanden werden, das/der vom Maulteil 101 weiter entfernt liegt als ein entsprechendes distales Element bzw. ein entsprechender proximaler Abschnitt.

Die Greifvorrichtung 100 umfasst weiterhin eine Zughülse 104, die mit einem proximalen Ende des Greifvorrichtungsdrahts 102 verbunden ist. Die Zughülse 104 ist an einem proximalen Ende 105 mit einem Federteller 106 verbunden (beispielsweise über ein Gewinde). An einem distalen Ende 107 der Zughülse 104 ist diese (gleitfähig) in einer Hülse 108 (Gewindehülse) und an diese proximal anschließend in einem zweiten Federteller 109 geführt. Zwischen dem ersten Federteller 106 und dem zweiten Federteller 109 ist ein erstes Federelement 110 (konkret eine Schraubenfeder) angeordnet.

Die Zughülse 104, der erste Federteller 106, der zweite Federteller 109 und das erste Federelement 110 sind wiederum in einer Ausnehmung 111 eines Übertragungselementes 112 gleitfähig gelagert. Das Übertragungselement 112 ist innerhalb der Griffschalen 8, konkret innerhalb von an dieser angeformten Griffschalenausnehmungen 32, gleitfähig gelagert. Zur gleitfähigen Lagerung des Übertragungselementes 112 dienen Übertragungselementzapfen 113, die in den schienenartigen Griffschalenausnehmungen 32 (siehe Fig. 2) geführt sind.

Weiterhin weist das Übertragungselement 112 einen Übertragungselementanschlag 114 (gegebenenfalls mehrere) auf, der bei einer Bewegung des Übertragungselementes 112 in proximale Richtung eine Kraft auf ein distales Ende des zweiten Federtellers 109 ausübt, der wiederum mittelbar über das erste Federelement 110, den ersten Federteller 106 und die Zughülse 104 eine Zugkraft auf den Greifvorrichtungsdraht 102 in proximale Richtung ausübt.

Die auf den Greifvorrichtungsdraht 102 wirkende Zugkraft führt zu einem Schließen des Maulteils 101, bis dieses entweder völlig geschlossen ist oder an ein zu greifendes Gewebe anliegt. Schwankungen in Gewebedicken können durch das Federelement 110 konstruktiv besonders einfach ausgeglichen werden, ohne dass das Gewebe zu locker oder zu fest gegriffen wird. Das Federelement hat somit auch eine Energiespeicherfunktion, so dass dem Anwender auf konstruktiv einfache Weise das Bedienen des Instrumentes erleichtert wird.

Eine Zugfeder 115 (konkret eine Schraubenzugfeder) ist einerseits an den Griffschalen 8 bzw. an diesen angeordneten Griffschalenstiften 33 aufgehängt und andererseits an einem proximalen Ende 116 des Übertragungselementes 112 derart, dass ein Verschieben des Übertragungselementes 112 in proximale Richtung eine Federkraft der Zugfeder 115 entgegenwirkt. Dadurch kann in der Position gemäß Fig. 2 und 3 besonders einfach eine selbsttätige Öffnung des Maulteils 101 gewährleistet werden. Dies erleichtert die Bedienung des elektrochirurgischen Instrumentes.

Die Schneidvorrichtung 200 umfasst ein Messer 201, das mit einer Schneidvorrichtungsstange oder einem Schneidvorrichtungsdraht 202 verbunden ist. Ein proximales Ende 203 des Schneidvorrichtungsdrahtes 202 ist (über eine Gewindehülse 204 und einen Stift 205) mit einem Transferelement 206 verbunden. Dies hat zur Folge, dass eine Verschiebung des Transferelementes 206 in distale Richtung über den Schneidvorrichtungsdraht 202 zu einer Verschiebung des Messers 201 in distale Richtung führt, so dass in dem Maulteil 101 gehaltenes Gewebe durchtrennt werden kann. In einer Transferelementausnehmung 207 ist ein zweites Federelement 208 angeordnet, wobei ein proximales Ende des zweiten Federelementes 208 an einem proximalen Ende des Transferelementes 206 anschlägt und ein distales Ende des zweiten Federelementes 208 an einen Stift 34 anschlägt, der ortsfest (beispielsweise integral angeformt) mit den Griffschalen 8 verbunden ist.

Die Griffschalen 8 bilden weiterhin ein Griffelement 35 aus, beispielsweise zur Aufnahme eines Daumens des Anwenders. Außerdem ist ein Drehelement 36 vorgesehen, mit dem das Maulteil 101 und/oder das Messer 201 verdreht werden kann.

In der Position des elektrochirurgischen Instrumentes gemäß den Fig. 2 und 3 kann durch eine Schließbewegung des Handabzuges 5 eine Kraft über eine Kniehebelvorrichtung 37 auf das Übertragungselement 112 übertragen werden, so dass dieses in proximale Richtung verschoben wird und das Maulteil 101 geschlossen wird. Die Kniehebelvorrichtung 37 umfasst eine (im Wesentlichen als Dreieck ausgeformte) Kniehebelplatte 38. Die Kniehebelplatte 38 umfasst eine erste Kniehebelplattenbohrung 39 und eine zweite Kniehebelplattenbohrung 40 sowie Kniehebelplattenstifte 41 (in Fig. 3 gestrichelt gezeichnet). In der ersten Kniehebelplattenbohrung ist ein ortsfest mit dem Handabzug 5 verbundener Handabzugsstift 42 geführt. In der zweiten Kniehebelplattenbohrung 40 ist ein ortsfest mit den Griffschalen 8 verbundener Griffschalenstift 43 geführt. Die (nach außen stehenden) Kniehebelplattenstifte 41 sind in einer Übertragungsplattenausnehmung 44 einer ersten Übertragungsplatte 45 sowie einer Übertragungsplattenausnehmung 46 einer zweiten Übertragungsplatte 47 geführt (gestrichelt gezeichnet). Die Übertragungsplattenausnehmung 44, 46 bzw. die Übertragungsplatten 45, 47 liegen in den Fig. übereinander. Die Übertragungsplatten 45, 47 weisen zweite Übertragungsplattenausnehmungen 48, 49 auf (in Fig. 3 gestrichelt gezeichnet), die durch die ortsfest mit dem Übertragungselement 112 verbundene Stifte 117 geführt sind.

Insgesamt umfasst die Kniehebelvorrichtung 37 somit ein erstes Kniehebelgelenk 50 umfassend die erste Kniehebelplattenbohrung 39 und den Handabzugsstift 42, ein zweites Kniehebelgelenk 51 umfassend die zweite Kniehebelplattenbohrung 40 und den Griffschalenstift 43, ein drittes Kniehebelgelenk 52 umfassend die Kniehebelplattenstifte 41 und die Übertragungsplattenausnehmungen 44, 46, und ein viertes Kniehebelgelenk 53 umfassend die zweiten Übertragungsplattenausnehmungen 48, 49 und die Stifte 117 des Übertragungselementes 112. Die Kniehebelgelenke 50-53 können auch abweichend von der hier dargestellten Konstruktion ausgebildet sein.

Das erste Kniehebelgelenk 50 ist ortsfest gegenüber dem Handabzug 5, das zweite Kniehebelgelenk 51 ist ortsfest gegenüber den Griffschalen 8, das vierte Kniehebelgelenk 53 ist ortsfest gegenüber dem Übertragungselement 112, wohingegen das dritte Kniehebelgelenk 52 gegenüber dem Handabzug 5, den Griffschalen 8 und dem Übertragungselement 112 beweglich ist.

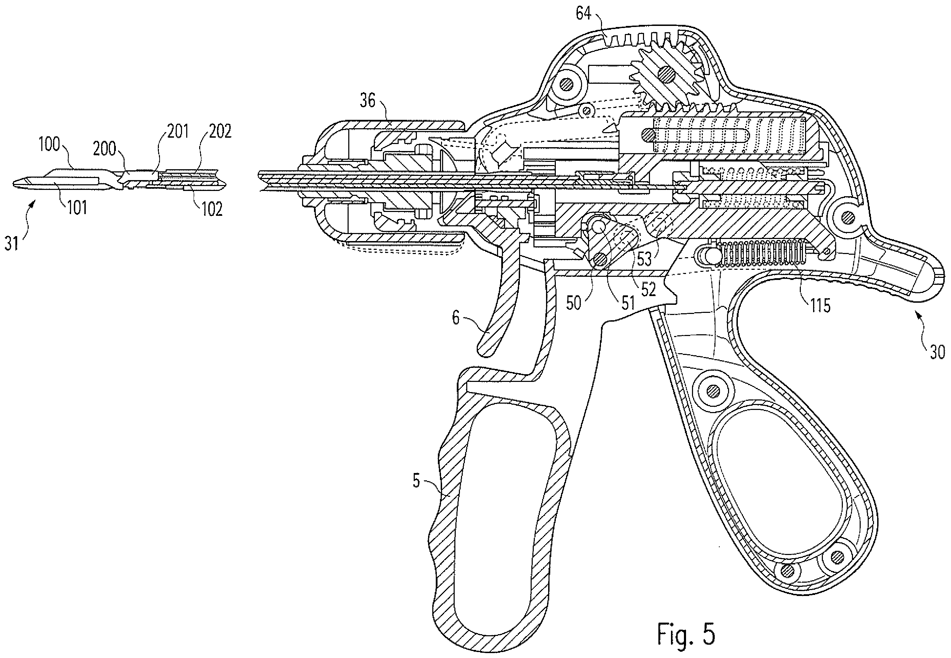

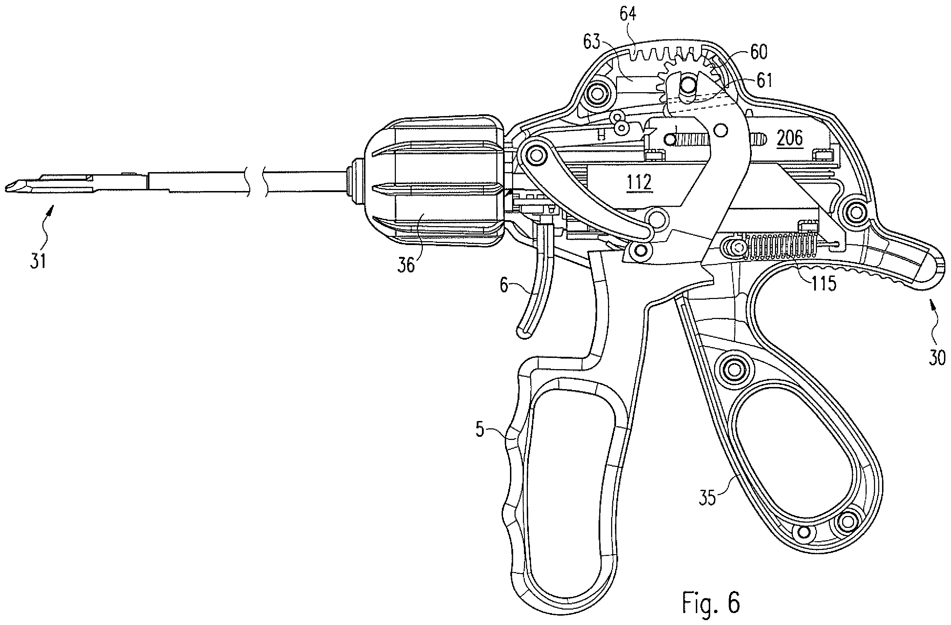

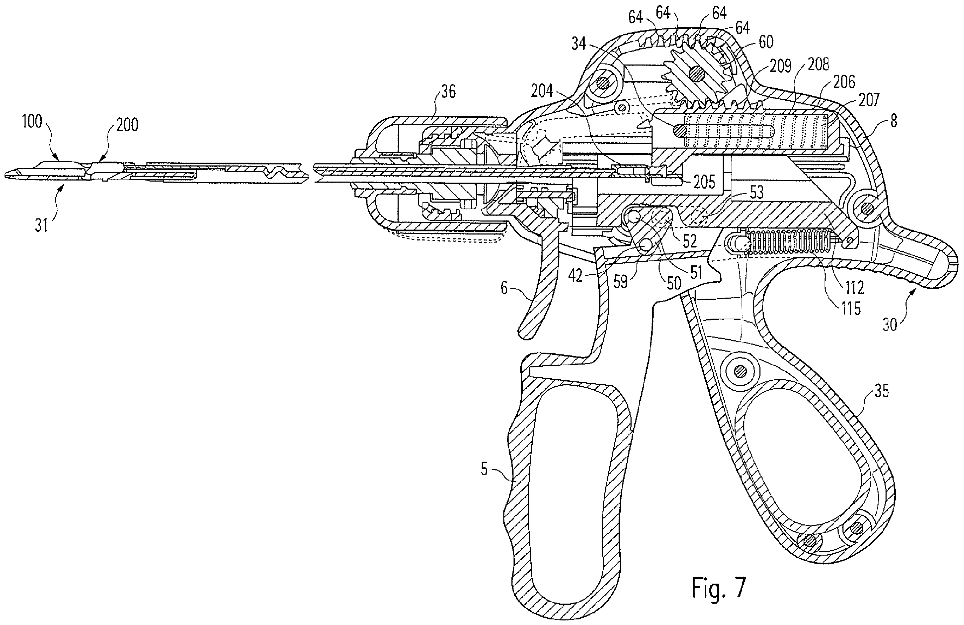

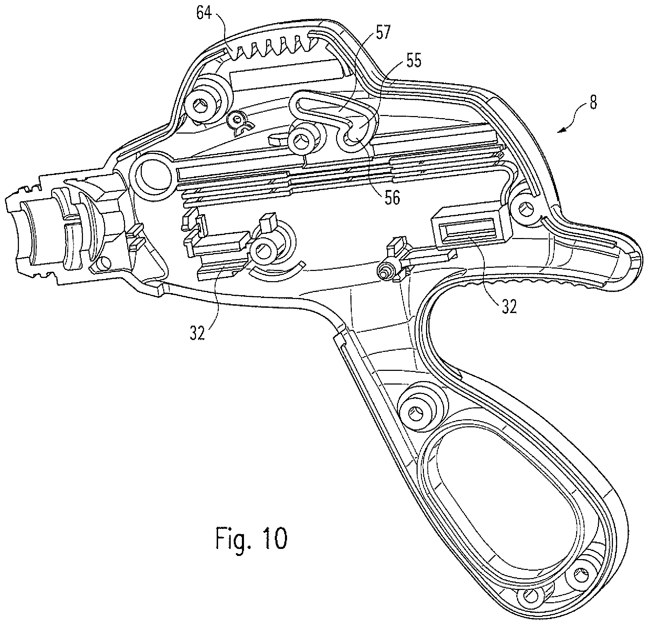

Der Handabzug 5 weist einen (oder mehrere) Handabzugsstift 54 (siehe Fig. 2) auf, der in einer V-förmigen Ausnehmung 55 mit einem kürzeren V-Schenkel 56 und einem längeren V-Schenkel 57 gehalten ist (siehe Fig. 10). In der Position gemäß Fig. 2 und 3 ist der Handabzugsstift 54 in dem kürzeren V-Schenkel 56 gehalten und wird durch diesen arretiert. Eine Schließbewegung des Handabzugs 5 führt dadurch zu einer Rotation des Handabzuges 5 um eine erste Rotationsachse 58 (siehe Fig. 2), die durch den Handabzugsstift 54 definiert wird. In Folge einer Rotation um die erste Rotationsachse 58 werden die Übertragungsplatten 45, 47 gegenüber der Kniehebelplatte 38 gestreckt (siehe Fig. 5), bis eine Totpunktstellung erreicht bzw. leicht überschritten wird (siehe Fig. 7) und die Kniehebelvorrichtung 37 arretiert ist. Der Totpunkt ist dabei überschritten, wenn das zweite, dritte und vierte Gelenk 51, 52, 53 in einer Linie angeordnet sind. In der Stellung gemäß Fig. 6 und 7 kann auch die Zugfeder 115 das Übertragungselement 112 nicht zurückführen. Ein zuverlässiges und vergleichsweise leicht einstellbares Greifen eines (nicht gezeigten) Gewebes ist somit auf einfache Weise möglich.

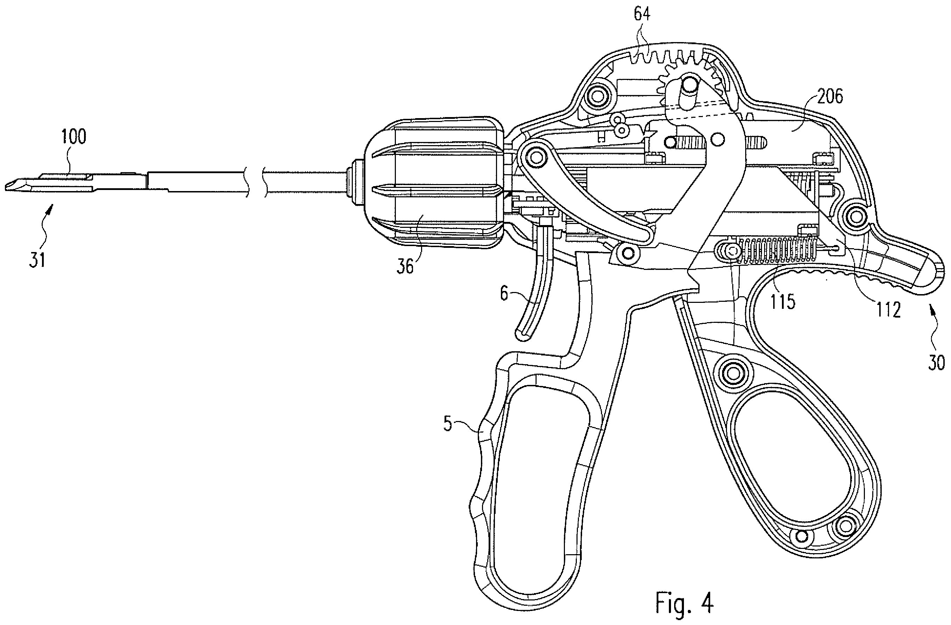

Der Totpunkt wird beispielsweise erreicht, wenn eine Verbindungslinie zwischen dem dritten und vierten Kniehebelgelenk 52, 53 auf eine Verbindungslinie zwischen dem ersten und zweiten Kniehebelgelenk 50, 51 (in etwa) senkrecht steht (diese Position ist eine Zwischenposition zwischen der Position gemäß Fig. 4 und 5 einerseits und 6 und 7 andererseits).

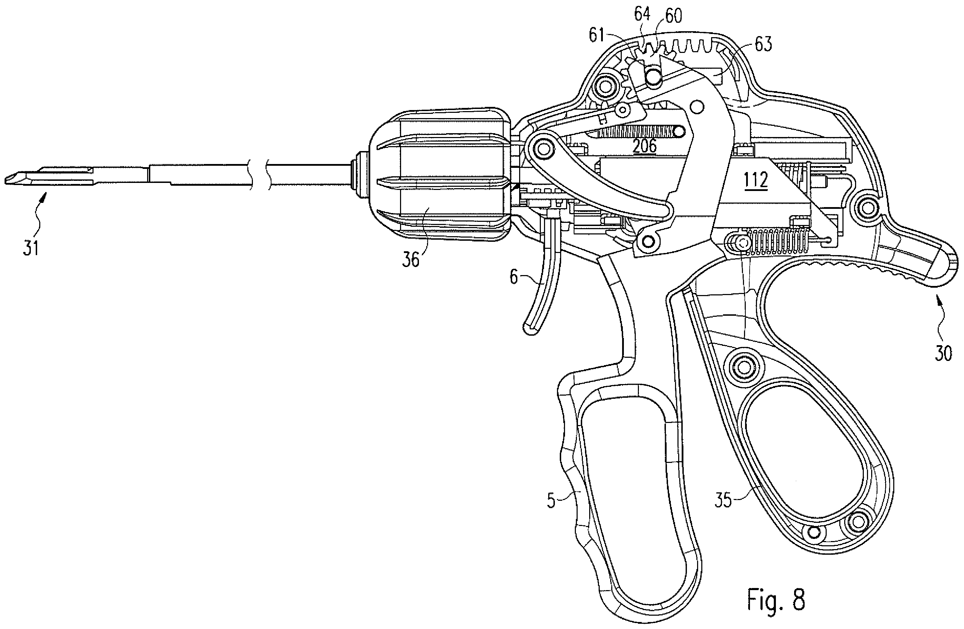

Die V-förmige Ausnehmung 55 ist derart angeordnet und ausgebildet, dass der Handabzugsstift 54 in der Position gemäß Fig. 7 und 8 nicht mehr arretiert ist (bzw. die Arretierung aufgelöst ist). Somit kann in der Position gemäß Fig. 7 und 8 der Handabzug 5 um eine zweite Rotationsachse 59 rotieren, die durch den Handabzugsstift 42 definiert ist. Ein weiteres Schließen des Handabzugs 5 führt dann zu einer Rotation des Handabzugs 5 um die zweite Rotationsachse 59, so dass ein Zahnrad 60, das innerhalb einer (radial verlaufenden, länglichen) Handabzugsausnehmung 61 gelagert ist, entlang einer länglichen Griffschalenausnehmung 63 (wobei an einer oder beiden Griffschalen 8 eine entsprechende Griffschalenausnehmung 63 vorgesehen sein kann) gleiten kann (in distale Richtung). Der Handabzugsstift 54 gleitet dabei in dem (leicht gekrümmten) V-Schenkel 57 der V-förmigen Ausnehmung 55. Um die leicht gekrümmte Bahn der V-förmigen Ausnehmung auszugleichen, ist die Handabzugsausnehmung 61 länglich ausgebildet, so dass das Zahnrad 60 in radialer Richtung innerhalb der Handabzugsausnehmung 61 verschiebbar ist.

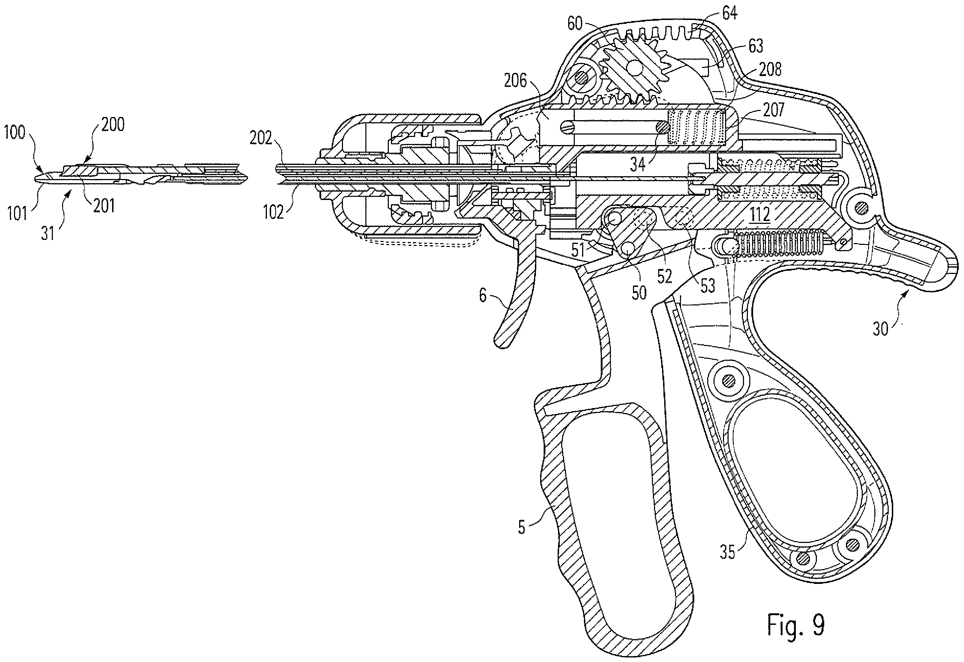

Ausgehend von der Position gemäß Fig. 6 und 7 wird somit das Zahnrad 60 in distale Richtung bis zu einer Endposition gemäß Fig. 8 und 9 innerhalb der länglichen Griffschalenausnehmung 63 verschoben. Durch das Vorsehen von Griffschalenzähnen 64 wird das Zahnrad 60 durch die Verschiebung in Rotation versetzt und kann das Transferelement 206, das Transferelementzähne 209 aufweist, ebenfalls in distale Richtung verschieben. Über den Schneidvorrichtungsdraht 202 wird somit das Messer 201 in distale Richtung zum Durchtrennen des Gewebes verschoben. Durch die Anordnung des Zahnrades 60 zwischen den Griffschalenzähnen 64 und den Transferelementzähnen 209 kann das Transferelement 206 durch eine vergleichsweise geringe Verschiebung des Zahnrades 60 bzw. eine vergleichsweise geringe Rotation des Handabzuges 5 um die zweite Rotationsachse 59, um eine vergleichsweise große Strecke verschoben werden. Dies erleichtert die Bedienung des elektrochirurgischen Instruments.

Die Stellung gemäß Fig. 6 und 7 auf der einen Seite und gemäß Fig. 8 und 9 auf der anderen Seite definieren zumindest einen Ausschnitt eines zweiten Teilbereichs eines Bewegungsbereichs des Handabzugs 5, bei dem ausschließlich die Schneidvorrichtung 200 betätigt wird. Innerhalb eines ersten Teilbereichs des Bewegungsbereichs, der durch die Positionen gemäß Fig. 2 und 3 auf der einen Seite und die Figuren 4 und 5 auf der anderen Seite zumindest ausschnittweise definiert ist, kann nur die Greifvorrichtung 100 bedient werden. Durch die Position des chirurgischen Instruments gemäß Fig. 4 und 5 auf der einen Seite und den Figuren 6 und 7 auf der anderen Seite wird ein Umkopplungsbereich definiert, innerhalb dessen beim Schließen des Handabzugs 5 eine Arretierung der Greifvorrichtung 100 hergestellt wird und eine Arretierung der Schneivorrichtung 200 aufgelöst wird.

Eine dritte Funktionseinheit, konkret eine Koagulationsfunktionseinheit, kann über das zweite Betätigungselement (den Fingerabzug) 6 separat vom Handabzug 5 bedient werden.

Die Greifvorrichtung 100 kann im ersten Teilbereich, die Schneidvorrichtung 200 im zweiten Teilbereich des Bewegungsteilbereichs beliebig hin und herbewegt werden, was die Bedienbarkeit weiter verbessert. Insbesondere beim Schneiden von Gewebe ist es vorteilhaft, wenn das Messer 201 zum Durchtrennen hin und herbewegt werden kann, was eine vergleichsweise glatte Schnittkante zur Folge hat. Auch beim Greifen von Gewebe ist gewährleistet, dass in jeder Position das Maulteil 101 weiter geöffnet oder weiter geschlossen werden kann. Alternativ ist es auch (abweichend von der gezeigten Ausführungsform) denkbar, weitere Rastmittel vorzusehen, die auch innerhalb des ersten bzw. zweiten Teilbereichs eine Arretierung erlauben, so dass bei bestimmten diskreten Positionen nur eine Bewegung in eine Richtung möglich ist.

Zum Greifen und Koagulieren von Gewebe dienen zwei Maulteilhälften des Maulteils 101. Im ersten Teilbereich können die Maulteilhälften geöffnet und geschlossen werden. Wird der Handabzug 5 gezogen, beispielsweise über eine Schließbewegung der Hand des Anwenders, wird über die Greifvorrichtung 100 das Maulteil 101 geschlossen. So kann Gewebe gegriffen und koaguliert werden. Das Koagulieren benötigt dabei nicht zwangsläufig eine mechanische Funktionseinheit.

Wird der Handabzug 5 über einen bestimmten Punkt hinausgezogen, verrastet die Greifvorrichtung 100 und das Maulteil 101 bleibt im geschlossenen Zustand. Zur Verrastung dient gemäß der konkreten Ausführungsform die Kniehebelvorrichtung 37. Befindet sich der Handabzug 5 im zweiten Teilbereich und treibt somit die Schneidvorrichtung 200 an, ist dieser vom ersten Teilbereich bzw. der Greifvorrichtung 100 entkoppelt. Wird der Handabzug 5 im zweiten Teilbereich weiter durchgezogen, bewegt die Schneidvorrichtung 200 das Messer 201 in distale Richtung durch das festgeklemmte Gewebe und durchtrennt dieses. Wird der Handabzug 5 wieder zurück in Richtung des Rastpunktes bewegt, wird das Messer 201 wieder zurückgeführt. Wird der Handabzug 5 über den Rastpunkt hinausgeführt, löst sich die Verrastung und die Maulteilhälften können geöffnet werden.

Im Allgemeinen ist die erste Funktionseinheit vorzugsweise durch Strecken des Kniehebelgelenkes und die zweite Funktionseinheit durch eine Rotation um die zweite Rotationsachse betätigbar. Dadurch erfolgt auf einfache Weise bei der Umkopplung ein Übergang von einer Zug- zu einer Schubbewegung, wobei der Anwender keinen Richtungswechsel bei der Betätigung durch das Betätigungselement durchführen muss, was die Handhabung des Instruments erleichtert.

Der Greifvorrichtungsdraht 102 und/oder der Schneidvorrichtungsdraht 202 sind vorzugsweise in einem Rohrschaft 68 geführt.

An dieser Stelle sei darauf hingewiesen, dass alle oben beschriebenen Teile für sich alleine gesehen und in jeder Kombination, insbesondere die in den Zeichnungen dargestellten Details, als Erfindung beansprucht werden. Abänderungen hiervon sind dem Fachmann geläufig.

### Bezuaszeichenliste

- 5: Betätigungselement (Handabzug)
- 6: zweites Betätigungselement (Fingerabzug)
- 7: Griffgehäuse
- 8: Griffschale
- 30: proximales Ende
- 31: distales Ende
- 32: Griffschalenausnehmung
- 33: Griffschalenstift
- 34: Stift
- 35: Griffelement
- 36: Drehelement
- 37: Kniehebelvorrichtung
- 38: Kniehebelplatte
- 39: erste Kniehebelplattenbohrung
- 40: zweite Kniehebelplattenbohrung
- 41: Kniehebelplattenstift
- 42: Handabzugsstift
- 43: Griffschalenstift
- 44: Übertragungsplattenausnehmung
- 45: erste Übertragungsplatte
- 46: Übertragungsplattenausnehmung
- 47: zweite Übertragungsplatte
- 48: zweite Übertragungsplattenausnehmung
- 49: zweite Übertragungsplattenausnehmung
- 50: erstes Kniehebelgelenk
- 51: zweites Kniehebelgelenk
- 52: drittes Kniehebelgelenk
- 53: viertes Kniehebelgelenk
- 54: Handabzugsstift
- 55: V-förmige Ausnehmung
- 56: kürzerer V-Schenkel
- 57: längerer V-Schenkel
- 58: erste Rotationsachse
- 59: zweite Rotationsachse
- 60: Zahnrad
- 61: Handabzugsausnehmung
- 63: längliche Griffschalenausnehmung
- 64: Griffschalenzahn
- 68: Rohrschaft
- 100: Greifvorrichtung
- 101: Maulteil
- 102: Greifvorrichtungsdraht
- 103: proximales Ende des Greifvorrichtungsdrahtes
- 104: Zughülse
- 105: proximales Ende der Zughülse
- 106: Federteller
- 107: distales Ende der Zughülses
- 108: Hülse
- 109: zweiter Federteller
- 110: erstes Federelement
- 111: Ausnehmung
- 112: Übertragungselement
- 113: Übertragungselementzapfen
- 114: Übertragungselementanschlag
- 115: Zugfeder
- 116: proximales Ende des Übertragungselementes
- 117: Stift
- 200: Schneidvorrichtung
- 201: Messer
- 202: Schneidvorrichtungsdraht
- 203: proximales Ende des Schneidvorrichtungsdrahtes
- 204: Gewindehülse
- 205: Stift
- 206: Transferelement
- 207: Transferelementausnehmung
- 208: zweites Federelement
- 209: Transferelementzahn

## Patentansprüche

1. Chirurgisches Instrument, mit einem Betätigungselement (5) zum Betätigen mindestens einer ersten (100) und einer zweiten Funktionseinheit (200), wobei das Betätigungselement (5) innerhalb eines Bewegungsbereichs rotatorisch bewegbar ist, wobei durch eine Bewegung innerhalb eines ersten Teilbereichs des Bewegungsbereichs die erste Funktionseinheit (100) betätigbar ist und durch eine Bewegung innerhalb eines zweiten Teilbereichs des Bewegungsbereichs die zweite Funktionseinheit (200) betätigbar ist,
wobei eine Umkopplungsvorrichtung vorgesehen ist derart, dass in Folge einer Bewegung des Betätigungselements (5) durch einen Umkopplungsbereich zwischen dem ersten und zweiten Teilbereich eine Wirkverbindung zwischen erster Funktionseinheit (100) und Betätigungselement (5) unterbrochen wird und eine Wirkverbindung zwischen zweiter Funktionseinheit (200) und Betätigungselement (5) hergestellt wird, wobei die Umkopplungsvorrichtung derart reversibel ausgebildet ist, dass eine Bewegungsumkehr zu einer in den Ausgangszustand zurückführenden Umkopplung führt, wobei erste und zweite Funktionseinheit (100,200) durch Betätigung des Betätigungselements (5) in jeder Position innerhalb des entsprechenden Teilbereiches in zwei verschiedene Richtungen bewegbar sind.

2. Chirurgisches Instrument, nach Anspruch 1,
**dadurch gekennzeichnet, dass**
mindestens eine Arretiervorrichtung vorgesehen ist derart, dass zumindest eine Teilkomponente der ersten Funktionseinheit (100) bei einer Bewegung des Betätigungselements (5) im zweiten Teilbereich arretiert ist.

3. Chirurgisches Instrument, nach Anspruch 1 oder 2,
**dadurch gekennzeichnet, dass**
die Arretiervorrichtung und/oder die Umkopplungsvorrichtung eine Kniehebelvorrichtung (37) umfasst.

4. Chirurgisches Instrument, nach einem der vorhergehenden Ansprüche,
**dadurch gekennzeichnet, dass**
die Umkopplungsvorrichtung und/oder die Arretiervorrichtung mindestens eine Führungsschiene umfasst, in der das Betätigungselement (5) führbar ist.

5. Chirurgisches Instrument, nach einem der vorhergehenden Ansprüche,
**dadurch gekennzeichnet, dass**
die erste/zweite Funktionseinheit eine Schneidvorrichtung (200) und/oder eine Greifvorrichtung (100) und/oder eine Festhaltevorrichtung und/oder eine Koagulationsvorrichtung und/oder eine Wasserstrahlvorrichtung und/oder eine optische Einrichtung und/oder eine Absaugvorrichtung, und/oder eine Spülvorrichtung und/oder eine Kryosonde und/oder Biopsievorrichtung und/oder eine Schneidelektrode umfasst.

6. Chirurgisches Instrument, nach einem der vorhergehenden Ansprüche,
**dadurch gekennzeichnet, dass**
mindestens eine Zahnstange und mindestens ein Zahnrad (60) zur Übertragung eine durch das Betätigungselement (5) aufgebrachte Kraft auf mindestens eine Funktionseinheit (100, 200) vorgesehen sind.

7. Chirurgisches Instrument, nach einem der vorhergehenden Ansprüche,
**dadurch gekennzeichnet, dass**
mindestens eine dritte Funktionseinheit durch ein weiteres Betätigungselement (6) betätigbar ist.

8. Chirurgisches Instrument, nach einem der vorhergehenden Ansprüche,
**dadurch gekennzeichnet, dass**
das Betätigungselement (5) einen Handabzug umfasst.

9. Chirurgisches Instrument, nach einem der vorhergehenden Ansprüche,
**dadurch gekennzeichnet, dass**
das Betätigungselement (5) um mindestens zwei voneinander beabstandete Rotationsachsen (58, 59) rotierbar ist.

10. Chirurgisches Instrument, nach einem der Ansprüche 3 bis 9,
**dadurch gekennzeichnet, dass**
eine/die erste/zweite Rotationsachse (58, 59) auf einer zentralen Achse der Kniehebelvorrichtung (37) liegt.

11. Chirurgisches Instrument, nach einem der vorhergehenden Ansprüche,
**dadurch gekennzeichnet, dass**
das Betätigungselement (5) rotierbar ist und eine Gleiteinrichtung translatorisch antreiben kann.

12. Chirurgisches Instrument, nach einem der vorhergehenden Ansprüche,
**dadurch gekennzeichnet, dass**
mindestens ein erstes Federelement (110) derart vorgesehen ist, dass eine Kraft des Betätigungselements (5) über das Federelement (110) an die erste/zweite Funktionseinheit (100, 200) übertragen wird.

13. Chirurgisches Instrument, nach einem der vorhergehenden Ansprüche,
**dadurch gekennzeichnet, dass**
das Betätigungselement derart ausgebildet ist, dass die Bewegung innerhalb des ersten Teilbereiches und/oder des zweiten Teilbereiches und/oder des Umkopplungsbereiches in einer Ebene durchführbar ist.

14. Chirurgisches Instrument, nach einem der Ansprüche 2 bis 13,
**dadurch gekennzeichnet, dass**
die Arretiervorrichtung derart reversibel ausgebildet ist, dass eine Bewegungsumkehr zu einer Aufhebung der Arretierung führt.

## Claims

1. Surgical instrument, having an actuating element (5) for actuating at least one first (100) and one second functional unit (200), wherein the actuating element (5) is movable in rotation within a movement range, wherein the first functional unit (100) is actuatable by means of a movement within a first partial range of the movement range and the second functional unit (200) is actuatable by means of a movement within a second partial range of the movement range,
wherein a coupling changeover device is provided such that, as a result of a movement of the actuating element (5) through a coupling changeover range between the first and second partial ranges, an operative connection between first functional unit (100) and actuating element (5) is interrupted and an operative connection between second functional unit (200) and actuating element (5) is produced, wherein the coupling changeover device is of reversible design such that a movement reversal leads to a coupling changeover that leads back to the initial state, wherein first and second functional unit (100, 200) are, by actuation of the actuating element (5), movable in two different directions in any position within the corresponding partial range.

2. Surgical instrument according to Claim 1,
**characterized in that**
at least one locking device is provided such that at least one partial component of the first functional unit (100) is locked during a movement of the actuating element (5) in the second partial range.

3. Surgical instrument according to Claim 1 or 2,
**characterized in that**
the locking device and/or the coupling changeover device comprises a knee lever device (37).

4. Surgical instrument according to one of the preceding claims,
**characterized in that**
the coupling changeover device and/or the locking device comprises at least one guide rail in which the actuating element (5) can be guided.

5. Surgical instrument according to one of the preceding claims,
**characterized in that**
the first/second functional unit comprises a cutting device (200) and/or a gripping device (100) and/or an immobilizing device and/or a coagulation device and/or a water jet device and/or an optical device and/or a suction device and/or a flushing device and/or a cryoprobe and/or a biopsy device and/or a cutting electrode.

6. Surgical instrument according to one of the preceding claims,
**characterized in that**
at least one toothed rack and at least one toothed wheel (60) are provided for transmitting a force that is imparted by the actuating element (5) to at least one functional unit (100, 200).

7. Surgical instrument according to one of the preceding claims,
**characterized in that**
at least one third functional unit is actuatable by means of a further actuating element (6).

8. Surgical instrument according to one of the preceding claims,
**characterized in that**
the actuating element (5) comprises a manually operated trigger.

9. Surgical instrument according to one of the preceding claims,
**characterized in that**
the actuating element (5) is rotatable about at least two mutually spaced-apart axes of rotation (58, 59).

10. Surgical instrument according to one of Claims 3 to 9,
**characterized in that**
a/the first/second axis of rotation (58, 59) lies on a central axis of the knee lever device (37).

11. Surgical instrument according to one of the preceding claims,
**characterized in that**
the actuating element (5) is rotatable and can drive a slide device in translation.

12. Surgical instrument according to one of the preceding claims,
**characterized in that**
at least one first spring element (110) is provided such that a force of the actuating element (5) is transmitted via the spring element (110) to the first/second functional unit (100, 200).

13. Surgical instrument according to one of the preceding claims,
**characterized in that**
the actuating element is designed such that the movement within the first partial range and/or the second partial range and/or the coupling changeover range can be performed in a plane.

14. Surgical instrument according to one of Claims 2 to 13,
**characterized in that**
the locking device is of reversible design such that a movement reversal leads to an elimination of the locking.

## Revendications

1. Instrument chirurgical comprenant un élément d'actionnement (5) destiné à actionner au moins une première unité fonctionnelle (100) et une deuxième unité fonctionnelle (200), l'élément d'actionnement (5) étant mobile en rotation dans une zone de déplacement, la première unité fonctionnelle (100) pouvant être actionnée par déplacement à l'intérieur d'une première sous-zone de la zone de déplacement et la deuxième unité fonctionnelle (200) pouvant être actionnée par un déplacement dans une deuxième sous-zone de la zone de déplacement,
un dispositif de recouplage étant prévu de telle sorte que, par suite d'un déplacement de l'élément d'actionnement (5) à travers une zone de recouplage située entre les première et deuxième sous-zones, une liaison fonctionnelle entre la première unité fonctionnelle (100) et l'élément d'actionnement (5) est interrompue et une liaison fonctionnelle entre la deuxième unité fonctionnelle (200) et l'élément d'actionnement (5) est établie, le dispositif de recouplage étant formé de manière à être réversible de telle sorte qu'une inversion du déplacement conduit à un recouplage de retour dans l'état initial, les première et deuxième unités fonctionnelles (100, 200) étant mobiles dans deux directions différentes par actionnement de l'élément d'actionnement (5) dans une position quelconque à l'intérieur de la sous-zone correspondante.

2. Instrument chirurgical selon la revendication 1, **caractérisé en ce que**
au moins un dispositif de blocage est prévu de telle sorte qu'au moins un sous-composant de la première unité fonctionnelle (100) est bloqué dans la deuxième sous-zone pendant un déplacement de l'élément d'actionnement (5).

3. Instrument chirurgical selon la revendication 1 ou 2, **caractérisé en ce que**
le dispositif de blocage et/ou le dispositif de recouplage comprend un dispositif à genouillère (37).

4. Instrument chirurgical selon l'une des revendications précédentes, **caractérisé en ce que**
le dispositif de recouplage et/ou le dispositif de blocage comprennent au moins un rail de guidage dans lequel l'élément d'actionnement (5) peut être guidé.

5. Instrument chirurgical selon l'une des revendications précédentes, **caractérisé en ce que**
la première/deuxième unité fonctionnelle comporte un dispositif de coupe (200) et/ou un dispositif de préhension (100) et/ou un dispositif de retenue et/ou un dispositif de coagulation et/ou un dispositif à jet d'eau et/ou un dispositif optique et/ou un dispositif d'aspiration et/ou un dispositif de rinçage et/ou une cryosonde et/ou un dispositif de biopsie et/ou une électrode de coupe.

6. Instrument chirurgical selon l'une des revendications précédentes, **caractérisé en ce que**
au moins une crémaillère et au moins une roue dentée (60) sont prévues pour transmettre une force, appliquée par l'élément d'actionnement (5), à au moins une unité fonctionnelle (100, 200).

7. Instrument chirurgical selon l'une des revendications précédentes, **caractérisé en ce que**
au moins une troisième unité fonctionnelle peut être actionnée par un autre élément d'actionnement (6).

8. Instrument chirurgical selon l'une des revendications précédentes,
**caractérisé en ce que**
l'élément d'actionnement (5) comprend un déclencheur manuel.

9. Instrument chirurgical, selon l'une des revendications précédentes, **caractérisé en ce que**
l'élément d'actionnement (5) peut tourner autour d'au moins deux axes de rotation (58, 59) distants l'un de l'autre.

10. Instrument chirurgical selon l'une des revendications 3 à 9, **caractérisé en ce que**
un/le premier/le deuxième axe de rotation (58, 59) est situé sur un axe central du dispositif à genouillère (37) .

11. Instrument chirurgical selon l'une des revendications précédentes, **caractérisé en ce que**
l'élément d'actionnement (5) est rotatif et peut entraîner en translation un mécanisme glissant.

12. Instrument chirurgical, selon l'une des revendications précédentes, **caractérisé en ce que**
au moins un premier élément à ressort (110) est prévu de telle sorte qu'une force de l'élément d'actionnement (5) est transmise à la première/deuxième unité fonctionnelle (100, 200) par le biais de l'élément à ressort (110).

13. Instrument chirurgical selon l'une des revendications précédentes, **caractérisé en ce que**
l'élément d'actionnement est conçu de telle sorte que le déplacement à l'intérieur de la première sous-zone et/ou de la deuxième sous-zone et/ou de la zone de recouplage peut être réalisée dans un plan.

14. Instrument chirurgical selon l'une des revendications 2 à 13, **caractérisé en ce que**
le dispositif de blocage est conçu de manière à être réversible de sorte qu'une inversion de déplacement entraîne une annulation du blocage.
